# EUROPEAN PATENT SPECIFICATION

(11) **EP 3 248 645 B1**
(45) Date of publication and mention of the grant of the patent: **24.07.2019**
(21) Application number: 17172814.0
(22) Date of filing: 24.05.2017
(51) Int. Cl.: A61F 2/07, A61B 17/12, A61M 25/10, A61F 2/966, A61M 25/00, A61M 31/00, A61F 2/06, A61F 2/82, A61F 2/915

(54) **DEVICE USEFUL FOR LOCALIZED THERAPEUTIC DELIVERY WITHOUT FLOW OBSTRUCTION**
VORRICHTUNG, DIE NÜTZLICH ZUR LOKALISIERTEN THERAPEUTISCHEN ABGABE OHNE FLUSSBEHINDERUNG IST
DISPOSITIF UTILE POUR UNE ADMINISTRATION THÉRAPEUTIQUE LOCALISÉE SANS OBSTACLE À L'ÉCOULEMENT

(30) Priority: 24.05.2016 US 201662340858 P
(43) Date of publication of application: 29.11.2017
(73) Proprietor: Cook Medical Technologies LLC, Bloomington, IN 47404 (US)
(72) Inventor: CERCHIARI, Alec, E., Bloomington, IN Indiana 47404 (US); NEFF, Gary L., Bloomington, IN Indiana 47408 (US); MAYLE, Brent A., Spencer, IN Indiana 47460 (US); MERK, James C., Terre Haute, IN Indiana 47802 (US); CHAMBERS, Sean D., Bloomington, IN Indiana 47401 (US); PAUL, JR.,, Ram H., Bloomington, IN Indiana 47403 (US); KIM, Seoggwan, West Lafayette, IN Indiana 47906 (US); SWIFT, Richard A., South Bend, IN Indiana 46635 (US)
(74) Representative: Simpson, Tobias Rutger

(56) References cited:
- EP-A1- 2 322 118
- WO-A2-95/08289
- US-A1- 2006 041 269
- US-A1- 2015 018 597

## Description

### REFERENCE TO RELATED APPLICATIONS

This application is a non-provisional application claiming the benefit of United States Provisional Patent Application No. 62/340,858, filed May 24, 2016.

### BACKGROUND

Many medical conditions are satisfactorily treated by the general systemic administration of a therapeutic agent. One drawback, however, associated with the systemic administration of therapeutic agents is that the systemically administered therapeutic agent may be absorbed not only by the tissues at the target site but by other areas of the body. Therefore areas of the body not needing treatment are also affected. Devices and methods for delivery of a therapeutic agent to only a selected portion of internal body tissue, without delivering the therapeutic agent to surrounding tissue or requiring additional systemic delivery of the therapeutic agent, are desired.

Medical delivery catheters provide a minimally invasive means for delivering therapeutic agents to internal body tissue. To provide site-specific localized treatment, balloon catheters may be used to deliver the therapeutic agent exclusively to the target site within a body vessel. One example of a condition that is beneficially treated by local administration of the therapeutic agent with a balloon catheter is the delivery of the therapeutic agent in combination with percutaneous transluminal coronary angioplasty (PTCA). Local administration of a therapeutic agent, however, is also beneficial in targeted chemotherapy, focal occlusion of vessels, thrombolysis, and targeted cell delivery, just to name a few examples.

During a site-specific therapy using existing balloon catheters, the catheter balloon is positioned at a target site, and the balloon is inflated, filling the vessel. The balloon is subsequently deflated and the catheter removed from the target site and from the patient's lumen thereby to allow fluid (e.g., body fluid) to flow freely through the lumen. Unfortunately, however, these balloon-based systems occlude blood flow and often cause distal ischemia, thereby limiting the window of time available for therapeutic delivery and their target sites for deployment. Therefore, there is a need for a tool that can deliver therapeutics, contrast agents, or biologics to a specific site within the human body without causing fluidic or gaseous occlusions. Perfusion balloon catheters exist; however, many allow only a small percentage of perfusion. For example, a balloon catheter sized for a 6 mm body vessel may only provide a 1 mm passageway.

In view of current devices and methods, there is a need for a medical device for applying vascular therapy locally within a body vessel while allowing fluid flow to areas distal to the treatment site.

With regard to the prior art, reference is directed to US2006/041269. The document discloses a vessel isolation device, which utilizes a reversible body partially covered by a blood impermeable sleeve. The device has a low profile collapsed state for delivery and a expanded state for deployment. The impermeable sleeve extends from a proximal portion to a distal portion of the expandable body and generally follows the contour of the expandable body. When deployed, the device is configured in the expanded state and has a generally hourglass, or dumbbell shape. In the expanded state, portions of the sleeve adjacent to the proximal and distal portions are placed in apposition to a vessel wall and blood is free to flow through the expandable body via inlets and outlets provided in the proximal and distal portions. As a result, an isolated treatment space is created that surrounds the device between the vessel wall and the blood impermeable sleeve adjacent to the neck portion of the expandable body.

In one embodiment, the vessel isolation device employs an expandable body constructed of struts, a sleeve, a catheter tube, and a deployment sheath. The catheter tube defines an access lumen and provides access to isolated treatment space after the vessel isolation device is deployed. The catheter tube passes through the proximal end of the vessel isolation device and is coupled to the sleeve so that the catheter's access lumen is in fluid communication with the isolated treatment space at an access port.

### SUMMARY

Aspects of the present invention are set out in the appended independent claims; selected embodiments are set out in the appended dependent claims.

Reference(s) to "embodiment(s)" throughout the description which are not under the scope of the appended claims merely represent possible exemplary executions and are therefore not part of the present invention.

In the following disclosure, medical devices and methods are provided. In some aspects, the present disclosure provides devices useful for applying therapy locally within a body lumen, such as a vascular vessel including arteries or veins while allowing flow through the vessel, the devices having a stent graft with flared end regions (e.g., a dog-bone stent graft) with a catheter providing fluid communication to the outer surface of the narrower, intermediate region of the stent graft. Specifically, in certain embodiments, the devices comprise a stent graft extending from a proximal end region to a distal end region and having an intermediate region positioned intermediate the proximal end region and the distal end region; the stent graft configurable from a contracted configuration to an expanded configuration; the stent graft defining a central lumen extending from the proximal end region to the distal end region in the expanded configuration and having a graft material portion in the intermediate region; the graft material portion having an inward-facing side that faces towards the central lumen and an outward-facing side that faces away from the central lumen; the proximal end region and the distal end region each having an average outer dimension when the stent graft is in the expanded configuration; the average outer dimensions, in the expanded configuration, of the proximal end region and the distal end region each being greater than an average outer dimension defined by the graft material of the intermediate region in the expanded configuration; and at least a first infusion/aspiration port in fluid communication with at least a first infusion/aspiration lumen; the first infusion/aspiration port opening on the outward-facing side of the graft material and the first infusion/aspiration lumen extending proximally from the first infusion/aspiration port along the proximal end region of the stent graft.

The proximal end region and/or the distal end region can be arranged to contact an inner surface of the body lumen wall to fluidly seal the intermediate region from other regions of the body lumen. The first infusion/aspiration lumen is preferably fluidly isolated from the central lumen. The stent graft can include a stent having an outward-facing side that faces away from the central lumen of the stent graft and an inward-facing side that faces towards the central lumen; and wherein the first infusion/aspiration lumen extends along the outward-facing side of the stent in the proximal end region. In some instances, a second infusion/aspiration port is in fluid communication with a second infusion/aspiration lumen; and the second infusion/aspiration port opens to the outward-facing of the graft material. In such instances, the first infusion/aspiration port and the second infusion/aspiration port can be diametrically and/or longitudinally opposed relative to the intermediate region of the stent graft. The first infusion/aspiration lumen and/or second infusion/aspiration lumen can be defined by one or more catheters, such as a bifurcated catheter. The infusion/aspiration lumens may have one or more infusion/aspiration ports per infusion/aspiration lumen.

The infusion/aspiration lumen can be positioned between the graft material and a second graft material with the second material bonded to the graft material. In some arrangements, the first infusion/aspiration extends proximally through a sheath and the sheath is sized and configured to contain the stent graft in the contracted configuration.

The average outer dimensions in the expanded configuration can be the average outer dimensions when the stent graft is expanded in its unconstrained condition. And, the proximal end region, the intermediate region, and the distal end region can each have an average outer dimension in the contracted configuration, and the average outer dimension of the proximal end region, the intermediate region, and/or the distal end region in the expanded configuration can be at least 20% greater than the average outer dimension of the same region in the contracted configuration. Preferably, the average outer dimension the region of highest expansion is at least 20% greater in the expanded configuration than in the contracted configuration. The proximal end region of stent graft can include a transitional portion and the transitional portion can have a portion positioned along a central, longitudinal axis of the stent graft when the stent graft is in an expanded configuration. In some instances, the transitional portion includes a helically-extending material (e.g., a spiral cut cannula).

Methods, not forming part of the invention, of using the devices of the present disclosure can include infusing a therapeutic agent through the first infusion/aspiration lumen towards the first infusion/aspiration port and out of the first infusion/aspiration port into contact with the outward-facing side of the graft material. Methods may include drawing a fluid contacting the outward-facing side of the graft material through the first infusion/aspiration port and into the first infusion/aspiration lumen. And, in some instances, methods include drawing a fluid contacting the outward-facing side of the graft material through a second infusion/aspiration port and into a second infusion/aspiration lumen; wherein the second infusion/aspiration port opens to the outward-facing side of the graft material. Advantageously, such arrangements can allow for continuous perfusion and/or aspiration of therapeutic agent. Additionally, such arrangements can help maintain high therapeutic concentrations and/or remove undesired side-products.

Further methods, also not forming part of the invention, of using the devices disclosed herein also include infusing a first liquid out of an infusion/aspiration port and into contact with the outward-facing side of the graft material and the first liquid transitioning into hydrogel form. In some instances, methods include infusing a first liquid out of an infusion/aspiration port and into contact with the outward-facing side of the graft material and infusing a second liquid out of an infusion/aspiration port and into contact with the first liquid so as to combine the first and second liquids. Such an arrangement may be useful for the combination of liquids inside a vessel of a patient, without the liquids reacting to one another or to the body of the patient prior to being exposed to the outward-facing side of the graft material.

The first and/or second liquids may include chemicals which combine to form a hydrogel in vivo for sealing or for regenerative applications. The following substances are contemplated as being infused and, when in contact with the outward-facing side for the graft material, transitioning from soluble liquid to hydrogel: hyaluronic acid, chitosan, alginate, poly(ethylene glycol), poly(N-isopropyl-acrylamide), proteins (e.g., collagen or fibrinogen), and/or peptides (e.g., RGD or IKVAV).

The transition from soluble liquid to hydrogel can occur spontaneously (i.e., physical self-assembly), through the action of chemical crosslinkers (i.e., catalyzed polymerization), and/or through irradiation (e.g., UV light irradiation). Self-assembly can take place through physical interactions that occur in response to temperature changes (e.g., from room temperature to body temperature) or time-sensitive changes in ionic and/or hydrophobic interactions. If hydrogel-formation occurs chemically, the liquid precursor reacts with another substance (e.g., liquid) that enables polymerization and, therefore, transition from soluble liquid to hydrogel upon mixing. The following cross-linkers may be used for polymerization: azide-alkyne(s), thiol-ene(s), diels-alder(s), oxime(s), and/or biologic(s) (e.g., thrombin). For hydrogel-formation through irradiation, the liquid precursor is irradiated, in many instances by light (e.g., UV light), causing the liquid to transition into hydrogel. In such instances, the device may be equipped with one or more emitters (e.g., light emitting diodes or fiber optic guides) arranged to irradiate the precursor liquid.

In arrangements that include the infusion of a first liquid and the infusion of a second liquid, the first and second liquids may be infused through separate infusion/aspiration lumens and/or ports. For example, the first liquid may be infused through a first infusion/aspiration lumen and a first infusion/aspiration port and the second liquid infused through a second infusion/aspiration lumen and a second infusion/aspiration port. However, it is contemplated that first and second liquids, in some instances, may be infused through the same infusion/aspiration lumen and/or infusion/aspiration port.

The present disclosure also provides kits including a stent graft, a catheter, and a sheath within a sterily sealed package; wherein the stent graft has a proximal portion, a distal portion, an intermediate portion positioned intermediate the proximal portion and the distal portion, and a stent configurable from a contracted configuration to an expanded configuration; wherein the stent graft includes a graft material portion extending along the intermediate portion; wherein the proximal portion and the distal portion each have an average outer dimension greater than an average outer dimension defined by the graft material portion in the intermediate portion; wherein the catheter communicates with a first infusion/aspiration port through a first infusion/aspiration lumen; wherein the first infusion/aspiration port opens to an outer side of the graft material of the stent graft; and wherein the stent graft and the catheter are positioned within a lumen of the sheath with the stent in the contracted configuration. In some instances, the kit includes a therapeutic agent in a container within the sterily sealed package.

Devices comprising a stent graft defining a central lumen extending from a proximal end region to a distal end region, the stent graft having an intermediate region positioned intermediate the proximal end region and the distal end region; and a first infusion/aspiration lumen extending along an outward-facing side of a graft material of the stent graft in the proximal end region and communicating with a first infusion/aspiration port is positioned in the intermediate region are also contemplated. In some instances, the stent graft of such devices includes a dog-bone shaped stent.

### BRIEF DESCRIPTION OF THE DRAWINGS

FIG. 1 is a perspective view of a device of the present disclosure in an expanded configuration.
FIG. 2 is a side view of a device of the present disclosure in an expanded configuration.
FIG. 3 is a side view of the device of FIG. 2 positioned within a vessel of the patient in the expanded configuration.
FIG. 4 is a cross-sectional view of a device of the present disclosure in an expanded configuration.
FIG. 5 is a cross-sectional view of a device in a contracted configuration in a delivery sheath.
FIG. 6 is a side view of a stent in a contracted configuration.
FIG. 7 is a side view of the stent of FIG. 6 in an expanded configuration.
FIG. 8 is a side view of a device positioned within a vessel of the patient in the expanded configuration.
FIG. 9 is a plan view of a device of the present disclosure.
FIG. 10 is a cross-sectional view taken along line 10-10 of FIG. 9.
FIG. 11 is a plan view of a kit of the present disclosure.
FIG. 12 is a cross-sectional view of a device of the present disclosure in an expanded configuration in a body vessel of a patient.
FIG. 13 is a side view of a device the present disclosure.
FIG. 14 is a side view of a device of the present disclosure.

### DESCRIPTION OF THE SELECTED EMBODIMENTS

For the purpose of promoting an understanding of the principles of the invention, reference will now be made to the embodiments illustrated in the drawings and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates. One embodiment of the invention is shown in great detail; although it will be apparent to those skilled in the relevant art that some features that are not relevant to the present invention may not be shown for the sake of clarity.

The language used in the claims and the written description and in the following definitions is to only have its plain and ordinary meaning, except for terms explicitly defined below. Such plain and ordinary meaning is defined here as inclusive of all consistent dictionary definitions from the most recently published (on the filing date of this document) general purpose Merriam-Webster dictionary.

As used in the claims and the specification, the following terms have the following defined meanings:
As used herein, the term "aspiration" means the withdrawal by suction. It includes but is not limited to the partial or complete removal of a material from a location of the body.

As used herein, the term "catheter" means an elongate medical device defining an internal lumen along a length thereof and configured for insertion into canals, vessels, passageways, or body cavities for diagnostic or therapeutic purposes. The term includes but is not limited to single-lumen and multi-lumen elongate medical devices having a length sufficient to extend outside of the body of the patient (e.g., 20cm or longer) and that are useful for the infusion or aspiration of fluid.

As used herein, the term "cells" includes endothelial cells, mesenchymoangioblasts, and bioengineering immune cells. For example, the cells can be cardiac muscle cells, lung cells, mesentery cells, or adipose cells. The adipose cells may be from omental fat, properitoneal fat, perirenal fat, pericardial fat, subcutaneous fat, breast fat, or epididymal fat. In certain embodiments, the cells comprise stromal cells, stem cells, or combinations thereof. Additional illustrative cells which can be used include hepatocytes, epithelial cells, Kupffer cells, fibroblasts, neurons, cardiomyocytes, myocytes, chondrocytes, pancreatic acinar cells, islets of Langerhans, osteocytes, myoblasts, satellite cells, endothelial cells, adipocytes, preadipocytes, biliary epithelial cells, and progentior cells of any of these cell types. The cells can include endothelial progenitor cells (EPCs) and/or muscle derived cells, including muscle derived myoblasts and/or muscle derived stem cells. The muscle derived cells can express desmin, M-cadherin, MyoD, myogenin, CD34, and/or Bcl-2, and can lack expression of CD45 or c-Kit cell markers. The term "cells" includes cellular populations of different types of cells, including adipose-derived stem and regenerative cells, sometimes also referred to as stromal vascular fraction cells, which can be a mixed population including stem cells, endothelial progenitor cells, leukocytes, endothelial cells, and vascular smooth muscle cells, which can be adult-derived. The "cells" (e.g., cellular preparation) can include adipose-derived cells that can differentiate into a nerve cell or a muscle cell. Suitable such cells and methods for obtaining them are described for example in U.S. Patent No. 6,777,231 and U.S. Patent No. 7,595,043.

As used herein, the term "endothelial progenitor cells" or "EPCs" include endothelial colony forming cells (ECFCs), especially ECFCs with high proliferative potential. Suitable such cells are described for example in U.S. Patent Application Publication No. 20050266556 published December 1, 2005, publishing U.S. Patent Application Serial No. 11/055,182 filed February 9, 2005, and U.S. Patent Application Publication No. 20080025956 published January 1, 2008, publishing U.S. Patent Application No. 11/837,999, filed August 13, 2007. Such ECFC cells can be a clonal population, and/or can be obtained from umbilical cord blood of humans or other animals. The endothelial colony forming cells can have the following characteristics: (a) express the cell surface antigens CD31, CD105, CD146, and CD144; and/or (b) do not express CD45 and CD14; and/or (c) ingest acetylated LDL; and/or (d) replate into at least secondary colonies of at least 2000 cells when plated from a single cell; and/or (e) express high levels of telomerase, at least 34% of that expressed by HeLa cells; and/or (f) exhibit a nuclear to cytoplasmic ratio that is greater than 0.8; and/or (g) have cell diameters of less than about 22 microns. Any combination of some or all of these features (a)-(g) may characterize ECFCs used in the present disclosure.

As used herein, the term "fluid" means a substance capable of flow and includes gasses and liquids.

As used herein, the term "infusion" means the introduction into a location of the body. It includes but is not limited to the introduction of a solution or suspension and may include introducing such a material into a vessel of a patient.

As used herein, the term "outer dimension" means the distance between outer surfaces in a plane orthogonal to the longitudinal axis of the stent graft. The term includes distances measured along radii of the stent graft.

As used herein, the term "stem cells" is used in a broad sense and includes traditional stem cells, adipose derived stem cells (e.g., cells derived from adipose tissue), progenitor cells, preprogenitor cells, reserve cells, and the like. Exemplary stem cells include embryonic stem cells, adult stem cells, pluripotent stem cells, neural stem cells, liver stem cells, muscle stem cells, muscle precursor stem cells, endothelial progenitor cells, bone marrow stem cells, chondrogenic stem cells, lymphoid stem cells, mesenchymal stem cells, hematopoietic stem cells, central nervous system stem cells, peripheral nervous system stem cells, and the like. Suitable such stem cells and methods for obtaining them are described, for example, in U.S. Patent No. 6,866,842 and U.S. Patent No. 7,155,417.

As used herein, the term "stent graft" means a device having a stent and a graft material (e.g., a covering material) extending along a portion of the stent. The stent graft may be tubular in shape. The stent may include a frame comprising or consisting of a biocompatible metal or metal alloy, such as stainless steel, nickel-titanium (e.g., Nitinol), gold, platinum, palladium, titanium, tantalum, tungsten, molybdenum, or alloys thereof. Other suitable alloys for the stent include cobalt-chromium alloys such as L-605, MP35N, and Elgiloy; nickel-chromium alloys, such as alloy 625; and niobium alloys, such as Nb-1% Zr, and others. Preferably, the stent is MRI-compatible and does not produce artifacts in images or scans obtained from magnetic resonance imaging. The stent may be fabricated from wire, tubing, or sheet using metal working and finishing techniques known in the art, such as drawing, extrusion, cold forming, gun drilling, laser welding, and laser cutting technologies. One or more of the stents of the stent graft may alternatively be made from a non-metallic material, such as a thermoplastic or other polymer. The stents may be designed to be either balloon-expandable or self-expanding. The material of the self-expanding stent preferably has shape memory/superelastic characteristics that enable it to "remember" and recover a previous shape. In the case of nickel-titanium shape memory alloys, the source of the shape recovery is a phase transformation between a lower temperature phase (martensite) and a higher temperature phase (austenite), which may be driven by a change in temperature (shape memory effect) and/or by the removal of an applied stress (superelastic effect). Strain introduced into the alloy in the martensitic phase to achieve a shape change may be substantially recovered upon completion of a reverse phase transformation to austenite, allowing the alloy to return to the previous shape. Recoverable strains of up to about 8-10% are generally achievable with nickel-titanium shape memory alloys. Other suitable shape memory alloys for the stent may include, for example, Cu-Zn-Al alloys and Fe-Ni-Al alloys. Polymer materials that may be suitable for the stent include polyether ether ketone (PEEK), polyethylene teraphthalate, polyurethane, polyamide, polyester, polyorthoester, polyanhydride, polyether sulfone, polycarbonate, polypropylene, high molecular weight polyethylene, polytetrafluoroethylene, or another biocompatible polymeric material, or mixtures or copolymers of these; polylactic acid, polyglycolic acid or copolymers thereof, a polyanhydride, polycaprolactone, polyhydroxy-butyrate valerate or another biodegradable polymer. Biodegradable metals such as magenesium and magnesium alloys are also contemplated. The graft material is a biocompatible material suitable as a barrier between body fluid and therapeutic agent and is preferably liquid impermeable. The graft material may be elastic and/or inelastic and/or may form a sleeve (e.g., tubular body). The graft material may be a continuous, unitary sheet of material or may comprise separate pieces of material. The graft material may comprise a woven or nonwoven sheet. The graft material can be pulled over the stent(s) and secured to structural components of the stent(s) by sutures, by loops of graft material, and/or by bonding with other material layers. Many different types of natural or synthetic graft materials may be employed. For example, the graft material may be formed in whole or in part from one or more silicones or polyesters, such as poly(ethylene terephthalate) or Dacron®; fluorinated polymers, such as polytetrafluoroethylene (PTFE) and expanded PTFE; polyurethanes; polypropylene; polyaramids; polyacrylonitrile; and/or nylons. Graft materials that are not inherently biocompatible may be suitable for use in the stent graft if they can be rendered biocompatible by, for example, surface modification techniques. Examples of surface modification techniques include graft material polymerization of biocompatible polymers from the material surface, coating of the surface with a crosslinked biocompatible polymer, chemical modification with biocompatible functional groups, and immobilization of a compatibilizing agent, such as heparin or other substances. It is also envisioned that the graft material may be impregnated or coated with one or more therapeutic drugs for release at the site of the aneurysm. The stent graft, or portions thereof, may be biodegradable.

As used herein, the term "therapeutic agent" means a substance useful in the treatment of a disease or disorder. It includes, but is not limited to small molecule drugs and contrast agents, nanoparticles, macromolecules, and cells. The term includes small molecule drugs useful for localized chemotherapy/oncology and/or vascular intervention such as dissolving thrombus and/or reducing vascular calcification. For example, drugs such as paclitaxel, rapamycin, myotropic/neurotropic antispasmodics, and anticalcificants such as phosphate binders are included. Contrast agents suitable for MRI, X-Ray, and/or ultrasound imaging are included, such as gadolinium, manganese, iron oxide, and iodine-based (ionic/non-ionic) contrast agents. Organic, inorganic, and/or complex/polymeric nanoparticles useful for thermal ablation and targeted drug-delivery are contemplated. This includes but is not limited to liposomes, micelles, perfluorocarbons, gold nanoparticles, superparamagnetic iron oxide nanoparticles (SPION), dendrimers and functionalized nanoparticles. Macromolecule proteins, peptides, and/or synthetic polymers useful for biochemical thrombectomy, cell adhesion, coercive morphogenesis, prolonged drug-release, and/or sealants are contemplated. This includes but is not limited to fibrinolytics (e.g., urokinase, tPA), adhesional proteins (e.g., Fn, Lama, Col), growth factors (e.g., VEGF, TGF, Insulin), drug-eluting gels, hydrogels and glues. Environmentally-responsive hydrogels that can transition from liquid to gel form at a desired temperature (e.g., at 37°C) and concentration are contemplated. Cells including differentiated, stem/progenitor, and/or genetically modified cells useful for re-endothelialization, endothelial regeneration and/or cellular therapy are contemplated as well as antisense and monoclonal antibodies.

Devices described herein include a hollow, dog-bone shaped, and self-expanding stent graft coupled with a catheter that allows for both infusion and aspiration (either simultaneously or independently) of a soluble cargo. Additionally, disclosed devices can be deployed within and subsequently retrieved from the target site. Advantageously, disclosed devices can isolate a segment of wall a body lumen, such as a vascular vessel, to locally deliver molecules or cells to the wall of the body lumen, an implanted medical device in the segment of the body lumen, and/or branch lumens of the body lumen without completely obstructing fluidic or gaseous flow through the body lumen.

It is contemplated that methods of using the devices disclosed but not forming part of the invention, may include adjusting the inflow and outflow of therapeutic agent into a target site based on the catalytic performance of the therapeutic agent in the target site. Additionally, it is contemplated that methods of using the devices disclosed herein but not forming part of the invention, to deliver cells may include adjusting the inflow of the suspension of cells and outflow of fluid (e.g., suspension of cells that did not adhere to the target site).

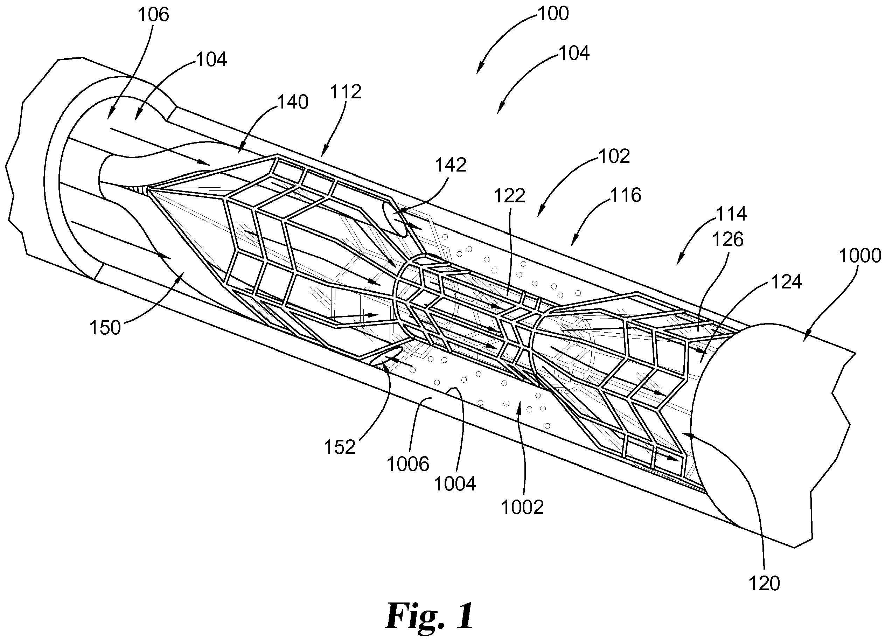

Figure 1 illustrates a perspective view of an exemplary device 100 of the present disclosure. The device comprises a stent graft 102, a catheter 104, and a delivery sheath 106. The stent graft has a proximal end region 112, a distal end region 114, and an intermediate region 116 positioned intermediate the proximal end region and the distal end region.

The stent graft is configurable from a contracted configuration to an expanded configuration. In the expanded configuration, the stent graft 102 defines a central lumen 120 extending along a length of the stent graft. The lumen is defined at least by a graft material 122 having an inward-facing side 124 that faces the central lumen 120 and an outward-facing side 126 that faces away from the central lumen 120 (i.e., towards the vessel wall 1006 when positioned within the body of a patient). The graft material 122 has a portion positioned at least in the intermediate region 116 of the stent graft and, in some instances, extends along the proximal end region 112 and/or the distal end region 114 of the stent graft. The graft material portion can be positioned on a radially inward-facing surface and/or a radially outward-facing surface of a stent of the stent graft.

The proximal end region 112, the intermediate region 116, and the distal end region 114 each have an average outer dimension when the stent graft 102 is in the expanded configuration. The expanded configuration may be the expanded configuration when the stent graft 102 is expanded in its unconstrained condition (e.g., without constraint from a sheath or vasculature) or expanded within a sheath or vasculature of a patient. The average outer dimension 130 of the proximal end region 112 and the average outer dimension 132 of the distal end region 114 are each greater than the average outer dimension 134 of the intermediate region 116 when the stent graft 102 is in the expanded configuration. When the stent graft 102 is positioned within a vessel 1000 of a patient, a space 1002 is defined between the intermediate region 116 of the stent graft and the inner surface 1004 of the vessel wall 1006. In some instances, the space 1002 is an annular space, extending circumferentially around the intermediate region 116 of the stent graft. The space 1002 may also be bounded at its proximal end by the proximal end region 112 and at its distal end by the distal end region 114.

The catheter of the device defines and/or communicates with a first infusion/aspiration lumen 140 communicating with at least a first infusion/aspiration port 142. A portion of the first infusion/aspiration lumen 140 (e.g., a portion of a catheter defining the infusion/aspiration lumen) extends along the proximal end region 112 of the stent graft and positions the first infusion/aspiration port 142 to open to the outward-facing surface of the graft material 126. In some instances, the first infusion/aspiration port 142 is positioned along or adjacent to the intermediate region 116 of the stent graft. The first infusion/aspiration port 142 provides fluid communication between the first infusion/aspiration lumen 140 and the space 1002 defined by the outward-facing surface of the graft material 126 in the intermediate region 116 of the stent graft and the inner surface 1004 of the vessel wall 1006 when the stent graft 102 is positioned in the expanded configuration within a vessel 1000.

In some instances, the catheter and/or first infusion/aspiration lumen 140 are positioned on the inward-facing surface of the graft material portion 124 in the proximal end region 112 and extend through an opening in the graft material 122 in the proximal end region 112 or intermediate region 116 to provide fluid communication between the first infusion/aspiration lumen 140 and a space on the outward-facing surface of the first material. In some instances, however, the catheter and/or first infusion/aspiration lumen 140 extend along a length of the proximal end region 112 on the outward-facing surface of the graft material portion 126 in the proximal end region 112 of the stent graft.

In some instances, the catheter includes a second infusion/aspiration lumen 150 and at least a second infusion/aspiration port 152 in fluid communication with the second infusion/aspiration lumen 150. Similar to the first infusion/aspiration lumen 140 and first infusion/aspiration port, the second infusion/aspiration lumen 150 can extend along the proximal end region 112 of the stent graft and position the second infusion/aspiration port 152 in fluid communication with the outward-facing surface of the graft material portion 126 in the intermediate region 116.

The first infusion/aspiration port 142 and/or second infusion/aspiration port 152 may open in a direction parallel to the longitudinal axis of the stent graft 190 or transverse to the longitudinal axis of the stent graft 190. For example, the infusion/aspiration port 142, 152 maybe angled away from the longitudinal axis of the stent graft 190, so as to face a vessel wall when implanted. Additionally or alternatively, the first infusion/aspiration port 142 and/or second infusion/aspiration port 152 may be defined by a tapered end of the catheter.

In some arrangements, the catheter has a proximal end region and a distal end region and a bifurcation of the catheter in the distal end region forms the first infusion/aspiration lumen 140 and the second infusion/aspiration lumen 150. In such instances, the first infusion/aspiration lumen 140 and second infusion/aspiration lumen 150 may be in fluid communication with a catheter lumen in the proximal end region of the catheter. However, in some instances, the first infusion/aspiration lumen 140 and the second infusion/aspiration lumen 150 may separately extend from the distal end region of the catheter to the proximal end region of the catheter without being in fluid communication with one another. As will be appreciated, the catheter may be a multi-lumen catheter and may include one or more additional lumens for infusion/aspiration and/or for receiving auxiliary devices such as a wire guide or microcatheter.

The first infusion/aspiration port 142, second infusion/aspiration port 152, first infusion/aspiration lumen 140, and/or second infusion/aspiration lumen 150 may be spaced from another around the circumference of the intermediate region 116 of the stent graft. For example, the first infusion/aspiration port 142 and second infusion/aspiration port 152 may be diametrically opposed relative to the intermediate region 116 of the stent graft. Advantageously, such an arrangement may provide for even distribution of the therapeutic agent in the space 1002 defined by the intermediate region 116 of the stent graft and the inner surface 1004 of the vessel wall 1006. Additionally, in some instances, the first infusion/aspiration lumen 140 and first infusion/aspiration port 142 are useful for the infusion of fluid (e.g., therapeutic agent) into the space 1002 around the intermediate region 116 of the stent graft (i.e., in fluid communication with the outward-facing surface of the graft material 126) and the second infusion/aspiration lumen 150 and second infusion/aspiration port 152 are useful for the withdrawal (e.g., aspiration) of fluid (e.g., blood and/or therapeutic agent) from the space 1002 defined by the intermediate region 116 of the stent graft and the inner surface 1004 of the vessel wall 1006, when the stent graft 102 is positioned within a vessel 1000.

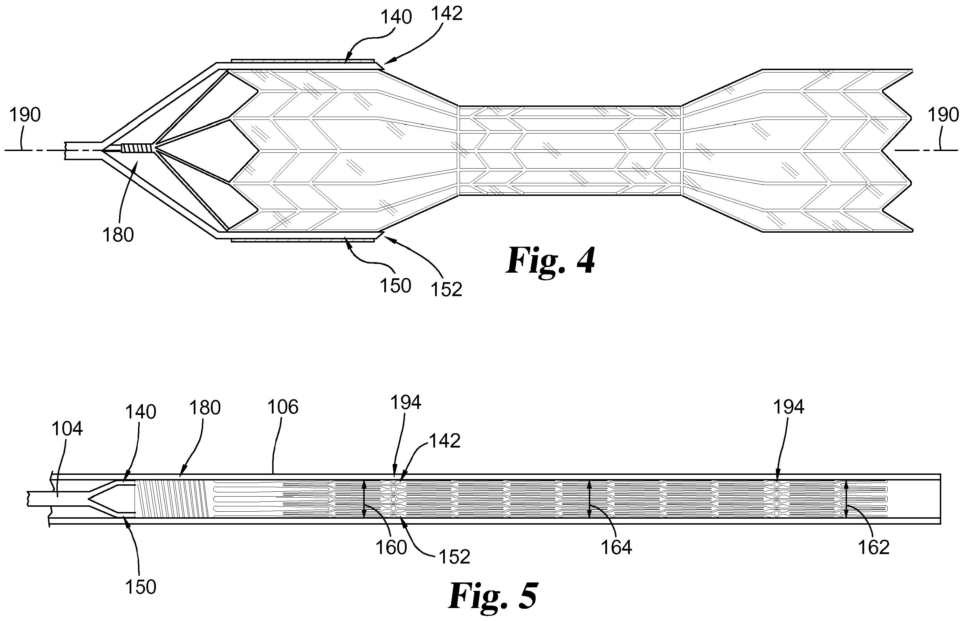

The catheter may be attached to the stent graft by one or more sutures, by bonding the catheter to the stent graft with a film and/or adhesive, and/or by molding a portion fo the stent graft in the catheter. For example, as illustrated in figure 4, the catheter may be positioned between a first graft material and a second graft material with the second graft material bonded to the first graft material and sandwiching the catheter there between. In some instances, the catheter is detachably connected to the stent graft 102, with the catheter being capable of detachment from the stent graft under a force of <10N or even <5N when the stent graft 102 is in the expanded configuration within the body of a patient. It is contemplated that the first infusion/aspiration lumen 140 and/or the second infusion/aspiration lumen 150 may be defined by opposing layers or plies of graft material extending along a length of the proximal end region 112 of the stent graft. In such instances, the first infusion/aspiration lumen 140 and/or the second infusion/aspiration lumen 150 may communicate with one or more catheters extending proximally of the proximal end region 112 of the stent graft. It is also contemplated that one or more infusion/aspiration lumens 140, 150 may be defined by a portion of the stent, such as a hollow strut.

In some arrangements, the device includes a sheath sized and configured to contain the stent graft 102 in the contracted configuration. This sheath may be arranged for advancement through the vasculature of a patient, such as by including a hydrophilic coating and/or rounded distal tip. Portions of the catheter are also contained within the sheath when the stent graft 102 is contained within the sheath in the contracted configuration.

In the contracted configuration, the proximal end region 112 of the stent graft has an average outer dimension 160, the distal end region 114 has an average outer dimension 162, and the intermediate region 116 has an average outer dimension 164. In some instances, the average outer dimension of the proximal end region 112, the intermediate region 116, and/or the distal end region 114 in the expanded configuration is at least 20% greater than the average outer dimension of the same region in the contracted configuration in the region of highest expansion. Additionally or alternatively, the proximal end region 112, the intermediate region 116, and the distal end region 114 each can have an average outer dimension in the contracted configuration of 7 mm or less.

Devices of the present invention may have multiple intermediate regions with the "intermediate region" referenced herein being one of the multiple intermediate regions. For example, any of the devices mentioned herein may include two or more intermediate regions, with at least one of the intermediate regions having a smaller outer dimension relative to one or more of the other intermediate regions.

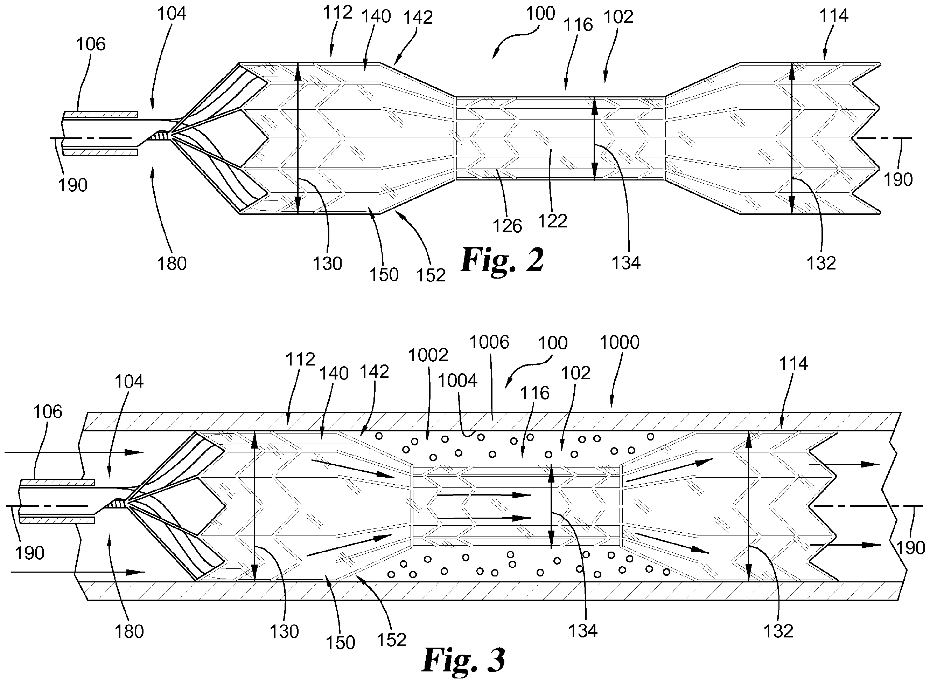

In some arrangements, the stent graft 102 includes a transitional portion arranged to increase flexibility in the proximal end region 112. As illustrated in figures 2-4, the transitional portion 180 can be positioned along a central, longitudinal axis 190 of the stent graft when the stent graft 102 is an expanded configuration. In some instances, as illustrated in figures 5, 6, and 7, the transitional portion includes a portion of helically-extending material (e.g., a spiral cut cannula). Advantageously, such an arrangement may aid in strain relief in the device, as taught in U.S. Publication No. 2016/0106405 and titled TRANSITIONAL GEOMETRY FOR AN EXPANDABLE MEDICAL DEVICE.

In some instances, a pushing member (e.g., a wire) and/or pulling member (e.g., a wire or string) may extend proximally from the transitional portion so as to be operable to selectively push the stent graft from a sheath and/or withdraw the stent graft into a sheath. In some instances, it is contemplated that the pushing member or pulling member may have a length sufficient to extend out of the body of the patient (e.g., a length of 30 cm or more). It is contemplated that, in some embodiments, the transitional portion may be configured and arranged to be grasped by a retrieval device, such as a retrieval loop, for the subsequent capture and withdrawal of the stent graft after deployment of the stent graft within the body of a patient.

The infusion/aspiration lumens 140, 150 may extend along an outward-facing surface of the transitional portion and/or along an inward-facing surface of the transitional portion. For example, in some instances, the first infusion/aspiration lumen 140 extends through the interior of the transitional portion.

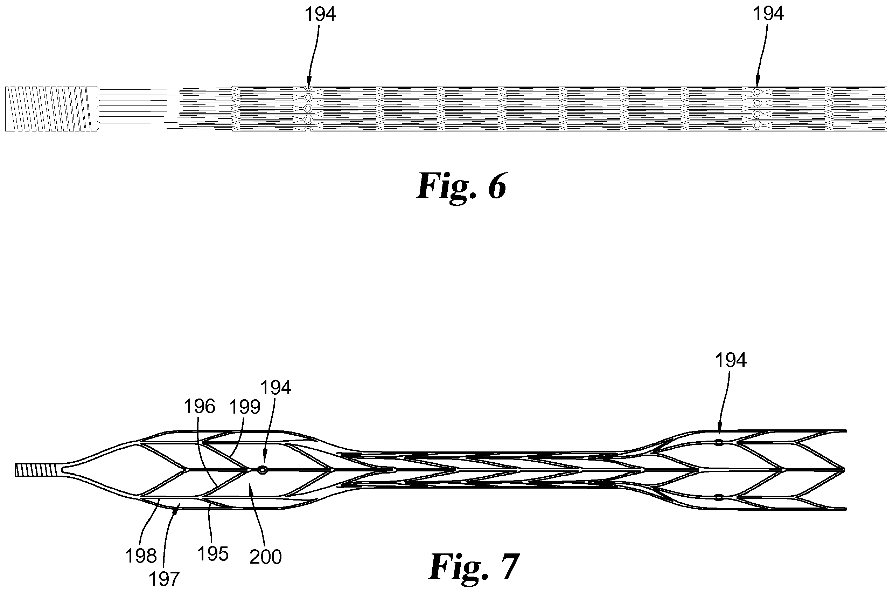

Figures 6 and 7 illustrate another embodiment of a stent for the stent grafts 102 disclosed herein. Figure 6 illustrates the stent in the contracted configuration, and figure 7 illustrates the stent in the expanded configuration. As will be appreciated, stents may be selected or designed to achieve a desired contracted configuration and expanded configuration.

The stent of any of the stent grafts disclosed herein may include a series of zig-zagging straight sections joined by bends, such as to form a plurality of serpentine rings. The stent of the stent graft 102 may be formed of an integral frame or a series of discrete frames along the length of the stent graft. For example, serpentine rings may be interconnected with longitudinal structural members and/or by securement of the rings to the graft material 122 of the stent graft 102. The stent may be fabricated from a cannula. In some instances, the stent may have longitudinal segments of laterally interconnected closed cells, as disclosed in U.S. Pat. Nos. 6,231,598, and 6,743,252.

In some embodiments, apices formed by intersecting struts and/or bends in struts of the stent are intersected by a longitudinally extending strut of the stent. For example, as shown in figure 7, proximal ends of strut 195 and strut 196 meet at a proximal apex 197, and apex 197 is intersected by longitudinal strut 198 that extends proximally from proximal apex 197. Similarly, distal ends of strut 196 and 199 meet at a distal apex 200 intersected by a longitudinal strut. Advantageously, having longitudinal struts intersecting and/or extending away from apices of the stent can aid in the delivery and/or retrieval of the stent graft 102 from a sheath. For instance, longitudinal strut 198 may help pull apex 197 inward (i.e., towards the longitudinal axis of the stent) as a sheath is advanced distally along the stent, thereby preventing the apex from becoming caught on the end of the sheath as the proximal end region of the stent is contracted and withdrawn into the sheath. Additionally, having apices positioned on longitudinally-extending members (e.g., longitudinal struts) can aid in manufacturing by improving the ease by which a stent may be advanced and/or withdrawn over a mandrel.

In some embodiments where the stent includes a plurality of undulating rings, such undulating rings may be arranged in-phase, as is shown in Figure 7, for example. In addition, or instead, where the undulating rings have similar shapes, generally longitudinally extending struts may connect together like points on adjacent undulating rings.

The struts of the stent grafts described herein may define apertures for one or more markers 194 (e.g., radiographic markers and/or echogenic markers) and/or for the infusion/aspiration lumens 140, 150. The markers may be arranged to indicate the position of the device or a portion of the device (e.g., the intermediate region 116) after the device has been inserted into the body of the patient. For example, markers may be positioned at the ends of the intermediate region 116 and/or at the proximal end and/or distal end of the graft material 122. Additionally, the markers may be arranged to indicate whether the stent graft 102 is in the expanded or contracted configuration.

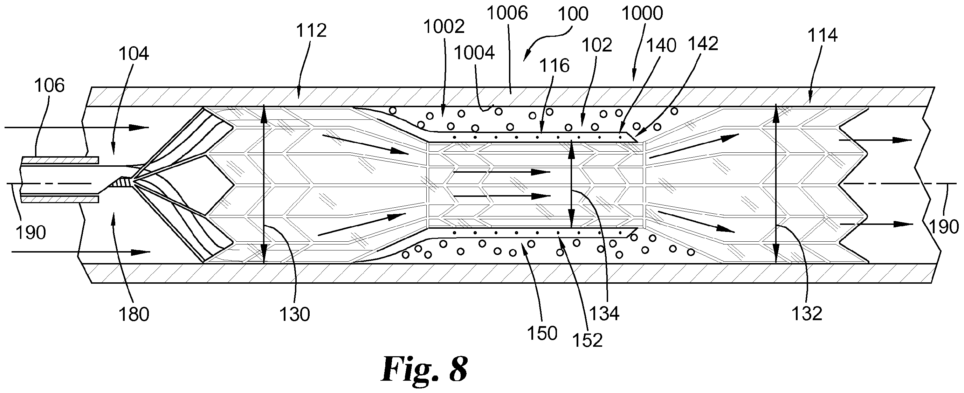

Figure 8 illustrates the infusion/aspiration lumens 140, 150 extending along the intermediate region 116. In some, but not necessarily all embodiments, the infusion/aspiration lumens 140, 150 have a plurality of openings/ports 152 for infusion/aspiration alongside the intermediate region 116. Such openings/ports 152 may, for example, be distributed along the length of the infusion/aspiration lumens 140, 150, as shown in Figure 8.

Such an arrangement may be used with any of the above-mentioned devices, including those in which the infusion/aspiration lumens 140, 150 extend along an outward-facing surface of the stent graft in the proximal end region 112. Similarly, as will be appreciated by those of skill in the art, any of the devices mentioned herein may have one or more lumens for infusion and one or more lumens for aspiration.

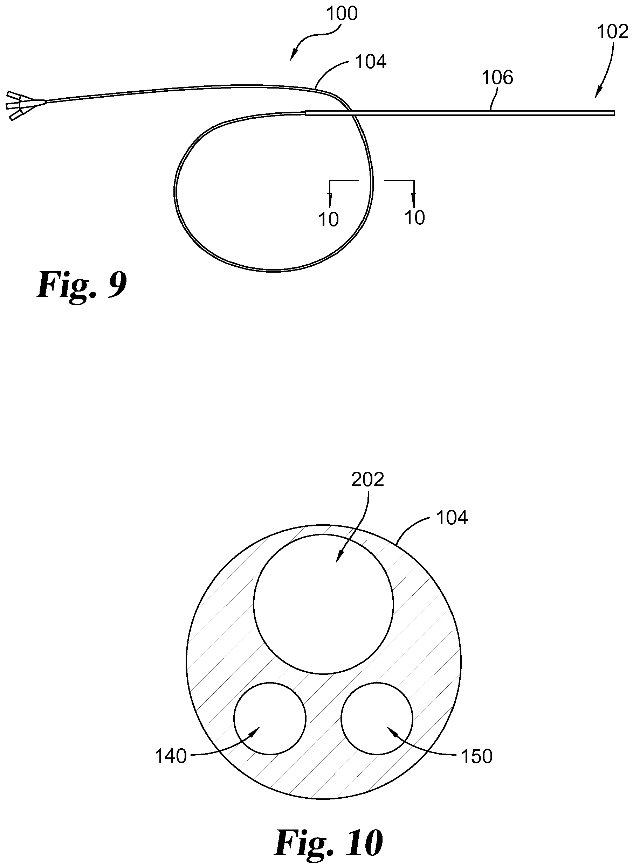

Figure 9 illustrates the device 100 with the stent graft 102 positioned within the sheath 106. Figure 10 illustrates a cross-sectional view of an embodiment of catheter 104. As discussed above, the catheter may include one or more lumens for the infusion and/or withdrawal of fluid to the space 1002 adjacent the intermediate region 116 of the stent graft on the outward-facing surface of the graft material 126. For example, the catheter may have a first infusion/aspiration lumen 140, a second infusion/aspiration lumen 150, and a guide wire lumen 202.

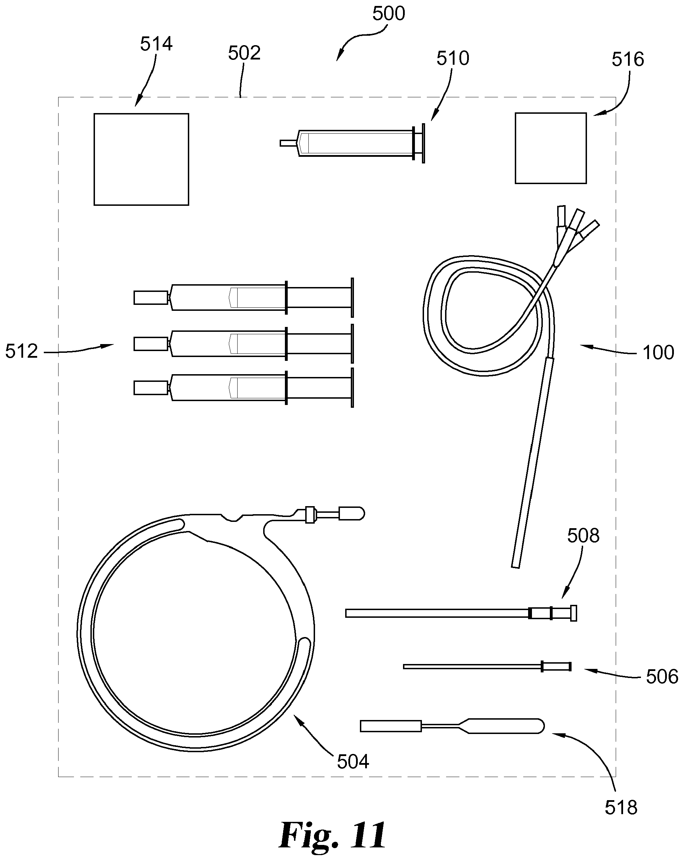

Kits (e.g., trays) containing the devices described above, and others, are contemplated. For example, as illustrated in figure 11, a kit 500 enclosed within sterily sealed medical package 502 may include a flexible-tip wire guide 504, an introducer needle 506, a dilator 508, an empty container (e.g., a vial or syringe) 510, a prefilled container 512, gauze sponges 514; a drape 516, a safety scalpel 518 and any of the devices mentioned above, including the devices illustrated in the accompanying figures.

A prefilled container (e.g., a vial or syringe) included in the kit 500 may contain a therapeutic agent useful with the above described devices. For example, a prefilled vial or syringe may include small molecule drugs useful for localized chemotherapy/oncology and/or vascular intervention such as dissolving thrombus and/or reducing vascular calcification. For example, drugs such as paclitaxel, rapamycin, myotropic/neurotropic antispasmodics, and anticalcificants such as phosphate binders may be included in the prefilled vials or syringes. Additionally or alternatively, contrast agents may be included in the solution or suspension contained within the prefilled vials and syringes. Contrast agents suitable for MRI, X-Ray, and/or ultrasound imaging are all contemplated, such as gadolinium, manganese, iron oxide, and iodine-based (ionic/non-ionic) contrast agents, just to name a few non-limiting examples.

Advantageously, nanoparticles may be included in the above kits and/or delivered using the above described devices. For example, organic, inorganic, and/or complex/polymeric nanoparticles useful for thermal ablation and targeted drug-delivery are contemplated. This includes but is not limited to liposomes, micelles, perfluorocarbons, gold nanoparticles, superparamagnetic iron oxide nanoparticles (SPION), dendrimers and functionalized nanoparticles.

Similarly, macromolecules may be included in the above kits and/or delivered using the above described devices. For example, proteins, peptides, and/or synthetic polymers useful for biochemical thrombectomy, cell adhesion, coercive morphogenesis, prolonged drug-release, and/or sealants are contemplated. This includes but is not limited to fibrinolytics (e.g., urokinase, tPA), adhesional proteins (e.g., Fn, Lama, Col), growth factors (e.g., VEGF, TGF, Insulin), drug-eluting gels, and glues.

It is also contemplated that cells may be included in the above kits and/or delivered using the above described devices. For example, differentiated, stem/progenitor, and/or genetically modified cells useful for re-endothelialization, endothelial regeneration and/or cellular therapy are contemplated. This includes but is not limited to endothelial cells, mesenchymoangioblasts, and bioengineering immune cells.

Systems incorporating the devices herein are also contemplated. For example, a system including a device disclosed herein and an apparatus for infusing/aspirating a fluid through one or more of the infusion/aspiration lumens 140, 150 of the device are contemplated. The apparatus or the system may include syringes and/or pumps. In some instances, the system may be configured to circulate a fluid through the infusion/aspiration lumen(s) of the device according to a treatment protocol. For example, the system may include one or more pumps configured to infuse therapeutic agent through the first infusion/aspiration lumen 140 into the space 1002 between the graft material 122 and the vessel wall 1006 and aspirate fluid from the same space 1002 using the second infusion/aspiration lumen 150. In some instances, the treatment protocol includes adjusting the inflow and outflow of therapeutic agent into a target site. This adjustment can be based on the catalytic performance of the therapeutic agent in the target site. Additionally, adjusting inflow and outflow of fluid can facilitate localization of therapeutics at the target site. It is contemplated that the treatment protocol may include adjusting the inflow of the suspension of cells and outflow of fluid (e.g., suspension of cells that did not adhere to the target site).

As mentioned above, the above devices may be useful for localized chemotherapy/oncology, vascular intervention such as dissolving thrombus and/or for reducing vascular calcification; for injecting contrast agents suitable for MRI, X-Ray, and/or ultrasound imaging; for thermal ablation and targeted drug-delivery; for biochemical thrombectomy, cell adhesion, coercive morphogenesis, prolonged drug-release, and/or sealants; and/or for endothelialization, endothelial regeneration and/or cellular therapy. It will be contemplated, however, that the above devices may be used for other therapies as well. For example, the above devices may be positioned over a medical device (e.g., a stent) and used to "treat" the medical device (e.g., promote endothelization of the medical device). It is also contemplated that the above device may be useful for sealing vessel dissections, such as aortic dissections. Such devices and/or methods may include use of a hydrogel sealant.

In one exemplary method which is not part of the invention, of using a device described herein, a distal end of a sheath containing the stent graft 102 of the device in a contracted configuration is advanced through a vessel of a patient towards a target location within the body of a patient. In some instances, the distal end of the sheath and the stent graft 102 are advanced percutaneously (i.e., through the skin of a patient). In other instances, the distal end of the sheath and the stent are advanced through a natural body opening (e.g., through the mouth and into the esophagus or trachea).

Once positioned at the target location within the patient, the sheath may be withdrawn and/or the stent graft 102 advanced so as to remove the stent graft 102 from within the sheath. After removal from the sheath, the stent graft 102 may be configured from the contracted configuration into the expanded configuration. For self-expanding stent grafts, the stent may self-expand into the expanded configuration. Advantageously, such self-expanding stent grafts can maintain juxtaposition with vessel walls as a function of vessel wall remodeling during a therapy (i.e., constriction or dilation). For balloon-expandable stent grafts, one or more balloons positioned within the lumen of the stent graft may be expanded so as to expand the stent graft into the expanded configuration.

In the expanded configuration, a space 1002 is defined between the outward-facing surface of the graft material 126 of the intermediate region 116 of the stent graft and the inner surface 1004 of the body vessel 1000. Additionally, in the expanded configuration, at least the first infusion/aspiration port 142of the first infusion/aspiration lumen 140 is in fluid communication with the above-mentioned space 1002. A fluid (e.g., a therapeutic agent in a solution or suspension and/or one or more liquid chemicals that can be formed into a hydrogel) may be infused through at least the first infusion/aspiration lumen 140 and out of the first infusion/aspiration port 142 into the space 1002, causing the fluid to come into contact with the outward-facing surface of the graft material 126 and with the inner surface 1004 of the vessel wall 1006. Multiple fluids may be infused and/or mixed when in contact with the outward-facing surface of the graft material 126. In some instances, those fluids are mixed to form a hydrogel. It is also contemplated that a fluid substance may be infused into contact with the outward-facing surface of the graft material 126 and then irradiated (e.g., irradiated with UV light) so as form a hydrogel and/or cure the infused substance or mixture.

In some instances, a fluid (e.g., blood) may be withdrawn from the space 1002 after expansion of the stent graft 102 into the expanded configuration and before and/or during infusion of a therapeutic agent. In embodiments having a second infusion/aspiration lumen 150 and a second infusion/aspiration port 152, a fluid maybe withdrawn and/or infused through the second infusion/aspiration lumen 150 before, during, and/or after the withdrawal and/or infusion of fluid through the first infusion/aspiration lumen 140. For example, in some instances, fluid is withdrawn from the space 1002 through the second infusion/aspiration lumen 150 while fluid is simultaneously being infused into the space 1002 from the first infusion/aspiration lumen 140 and first infusion/aspiration port 142. Advantageously, such an arrangement can allow for the removal of cells that did not adhere to the target site while supplying new viable cells.

Advantageously, the above infusion and/or withdrawal of fluid while the stent graft 102 is in the expanded configuration may occur while body fluid is capable of flowing through the lumen of the stent graft. After infusion and/or withdrawal of fluid through the first infusion/aspiration lumen 140and/or second infusion/aspiration lumen 150, the stent graft 102 may be selectively retrieved, such as by withdrawing the transitional portion into the sheath, contracting the stent graft 102 into the contracted configuration, and with drawing the stent graft 102 and the sheath from the body of the patient. Retrieval may be accomplished with the stent graft 102 either attached or detached from the catheter.

In some instances, the catheter is detached from the stent graft 102 after infusion and/or withdrawal of fluid through at least the first infusion/aspiration lumen 140 and first infusion/aspiration port 142. In such instances, the catheter may be detached from the stent graft 102 and the stent graft 102 left in place (e.g., implanted in the patient) permanently or temporarily (e.g., for a period of minutes, hours, days, weeks, or months).

Advantageously, the above devices can allow for a localized procedure within a vessel of a patient without substantial occlusion of the vessel during the treatment. In some instances, the average cross-sectional area of the lumen (measured perpendicular to the longitudinal axis 190) defined by the intermediate region 116 of the stent graft is at least 25% of the average cross-sectional area of the lumen defined by the proximal end region 112 and/or distal end region 114. In some embodiments, the average cross-sectional area of the lumen defined by the intermediate region of the stent graft is at least 50% or at least 75% of the average cross-sectional area of the lumen defined by the proximal end region 112 and/or distal end region 114. In some instances, the average outer dimension 134 of the intermediate region 116 may be at least 25% to 75% the average outer dimension of the proximal end region 130 and/or the distal end region 132. In some embodiments, the average outer dimension of the intermediate region 134 is approximately 50% the average outer dimension of the proximal end region 130 and/or the distal end region 132.

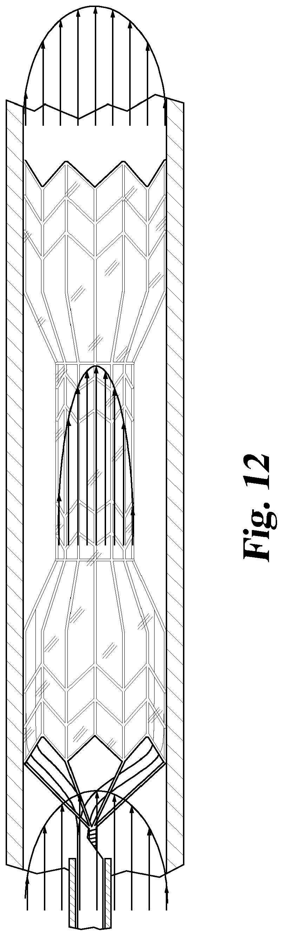

As illustrated in figure 12, the graft material 122 of the stent graft 102 separates the therapeutic agent provided to the target area from the bodily fluid flowing through the body vessel, with the therapeutic agent on one side of the graft material 122 and the bodily fluid on the other side of the graft material 122. In many embodiments, an inside dimension of the stent graft will be greater in the proximal end region 112 and/or distal end region 114 of the stent graft than in the intermediate region, resulting in the velocity of the bodily fluid flowing through the body vessel and the stent graft 102, illustrated by the velocity curves in figure 12, to be greater in the intermediate region 116 of the stent graft than in the proximal end region 112 and/or distal end region 114.

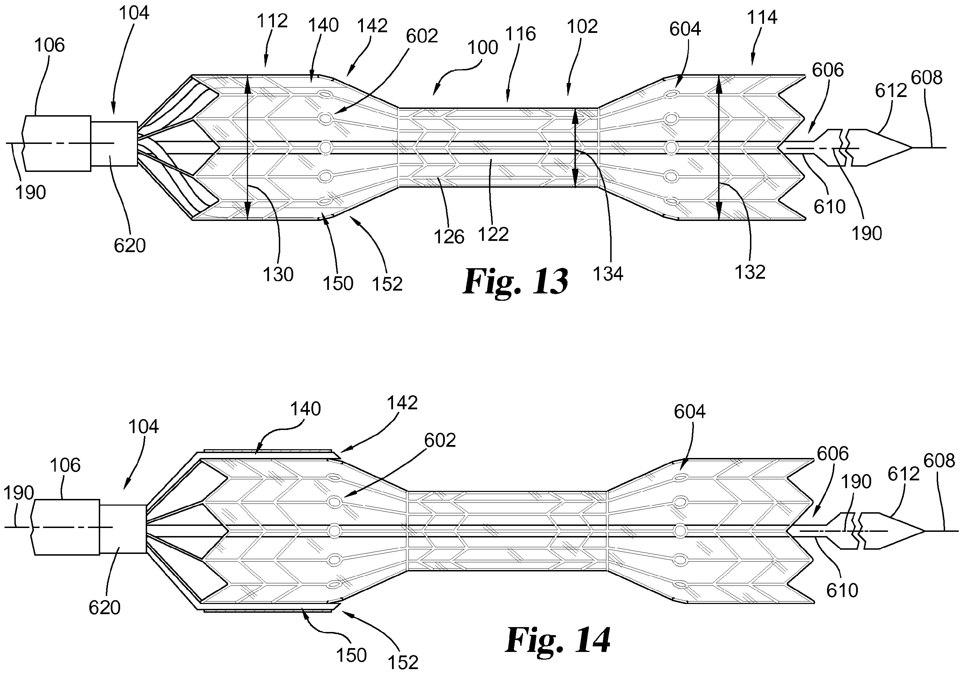

The embodiments described herein may include one or more radiopaque markers. For example, embodiments of device 100 may have a proximal radiopaque marker 602 and a distal radiopaque marker 604, as illustrated in figures 13 and 14. The proximal radiopaque marker can be positioned in the proximal end region 112, and the distal radiopaque marker can be positioned in the distal end region 114.

In some instances, the proximal radiopaque marker is positioned at or near a distal end of proximal end region 112 (e.g., adjacent intermediate region 116). Similarly, in some arrangements, the distal radiopaque marker is positioned at or near a proximal end of distal end region 114 (e.g., adjacent intermediate region 116). In this way, the proximal and/or distal radiopaque markers can indicate the proximal and/or distal boundaries of the intermediate region 116 when visualized with a medical imaging system, such as x-ray or ultrasound. Advantageously, this can indicate the area in the vessel that will receive infusate from one or more fluid infusion/aspiration lumens (e.g., first infusion/aspiration lumen 140 and/or second infusion/aspiration lumen 150) during an infusion procedure.

More or fewer radiopaque markers than those described above and illustrated in the figures are contemplated. For example, in some embodiments, device 100 can have a proximal radiopaque marker positioned at or near the proximal end of the stent and/or the graft material 122 (e.g., covering material) in the proximal end region 112 of the device. Similarly, the device can have a distal radiopaque marker positioned at or near the distal end of the stent and/or the graft material 122 (e.g., covering material) in the distal end region 114 of the device. Advantageously, such markers can indicate the sealing regions of the stent graft so as to help avoid the covering material in the proximal end region 112 and/or distal end regions 114 being inadvertently positioned over a branch vessel during a procedure.

Embodiments described herein may also include guidewire lumen for receiving a guidewire. The guidewire lumen may be defined by a catheter segment that extends through the central lumen of the stent graft. In some arrangements, the guidewire lumen may terminate at a distal end positioned distal of the distal end region 114 of the stent graft. For example, the guidewire lumen may be defined by a catheter segment that extends beyond the distal-most end of the stent graft. In many instances, the catheter segment defining the guidewire lumen includes a dilator tip at the distal end.

Both figures 13 and 14 illustrate a guidewire lumen 606 for receiving a guidewire 608, as described above. Figure 13 illustrates an embodiment having an infusion/aspiration lumen 140, 150 positioned radially within the stent in the proximal end region 112 of the stent graft, and figure 14 illustrates an embodiment having an infusion/aspiration lumen positioned 140, 150 radially outward of the stent in the proximal end region 112 of the stent graft.

The catheter segment defining the guidewire lumen 606 may be a segment of the same catheter defining the first and/or second infusion/aspiration lumens 140, 150. For example, the guidewire lumen 606, the first infusion/aspiration lumen 140, and/or the second infusion/aspiration lumen 150 may be lumens of a single catheter (e.g., a dual-lumen or tri-lumen catheter). Alternatively, the catheter segment defining the guidewire lumen may be a separate catheter from a catheter defining the first and/or second infusion/aspiration lumens 140, 150.

The embodiments described herein may also include a pusher 620 arranged to push the device from the delivery sheath. The pusher may be coupled to the stent graft 102 and/or catheter segments defining the first infusion/aspiration lumen 140, second infusion/aspiration lumen 150, and/or guidewire lumen. In many instances, the pusher is arranged to pull the stent graft 102 into the delivery sheath.

The stent graft 102 of any of the embodiments described herein may be a self-expanding stent graft or a balloon expandable stent graft. In some arrangements, one or more expandable balloons may be positioned inside the proximal and distal end regions 112, 114 of the stent graft. For example, a first expandable balloon may be positioned inside the proximal end region 112 of the stent graft and a second expandable balloon may be positioned inside the distal end region 114 of the stent graft. In some instances, an interior of an expandable balloon portion positioned inside the proximal end region 112 of the stent graft is in fluid communication with an interior of an expandable balloon portion positioned inside the distal end region 114 of the stent graft. In some instances, the portion of the central lumen within the intermediate region 116 of the stent graft is free of an expandable balloon.

Expandable balloons for expanding portions of the stent graft are in fluid communication with one or more inflation/deflation lumens defined by one or more catheter segments. The one or more catheter segments may be a portion of a catheter defining the guidewire lumen, first infusion/aspiration lumen 140, and/or second infusion/aspiration lumen 150, or the one or more catheter segments may be a portion of a separate catheter. Advantageously, in some embodiments, the expandable balloon(s) are removable from inside the stent graft 102 after the stent graft 102 is expanded to the expanded configuration. For example, the expandable balloon(s) may be provided on a balloon catheter that is removably positioned in the central lumen of the stent graft.

The delivery sheath mentioned in any embodiment described herein may be a splittable sheath. Such sheaths are also referred to as "peel away" sheaths and are capable of being split along their length so as to allow removal of a catheter and/or guide wire positioned within the lumen of the sheath without movement of the sheath over an end of the catheter and/or guidewire.

## Claims

1. A device, comprising:
a stent graft (102) extending from a proximal end region (112) to a distal end region (114) and having an intermediate region (116) positioned intermediate said proximal end region (112) and said distal end region (114);
said stent graft (102) configurable from a contracted configuration to an expanded configuration;
said stent graft (102) defining a central lumen (120) extending from the proximal end region (112) to the distal end region (114) in said expanded configuration and having a graft material (122) portion in said intermediate region;
said graft material (122) portion having an inward-facing side (124) that faces towards said central lumen (120) and an outward-facing side (126) that faces away from said central lumen (120);
said proximal end region (112) and said distal end region (114) each having an average outer dimension when said stent graft (102) is in said expanded configuration;
said average outer dimensions, in said expanded configuration, of said proximal end region (112) and said distal end region (114) each being greater than an average outer dimension defined by said graft material (122) of said intermediate region in said expanded configuration; and
a first infusion/aspiration port (142) in fluid communication with a first infusion/aspiration lumen (140);
said first infusion/aspiration port (142) opening on said outward-facing side (126) of said graft material (122), and said first infusion/aspiration lumen (140) extending proximally from said first infusion/aspiration port (142) along said proximal end region (112) of said stent graft, and extending along an outward-facing side of a graft material of said stent graft in said proximal end region (112).

2. The device of claim 1, wherein said stent graft includes a stent having an outward-facing side that faces away from the central lumen of the stent graft and an inward-facing side that faces towards the central lumen; and
wherein said first infusion/aspiration lumen extends along said inward-facing side of said stent in said proximal end region.

3. The device of any preceding claim, wherein said stent is self-expanding.

4. The device of any preceding claim, wherein a second infusion/aspiration port is in fluid communication with a second infusion/aspiration lumen; and
wherein said second infusion/aspiration port opens to said outward-facing side of said graft material;
preferably wherein said infusion/aspiration ports are spread evenly around said intermediate region of said stent graft.

5. The device of claim 4, wherein said first lumen is configured as an infusion lumen, said first port is configured as an infusion port, said second lumen is configured as an aspiration lumen and said second port is configured as an aspiration port.

6. The device of any preceding claim, wherein said first infusion/aspiration lumen is defined by a catheter.

7. The device of any preceding claim, wherein said first infusion/aspiration lumen is positioned between said graft material and a second graft material; and
wherein said second graft material is bonded to said graft material.

8. The device of any preceding claim, wherein said infusion/aspiration lumen extends in a proximal direction through a sheath; and
wherein said sheath is sized and configured to contain said stent graft in said contracted configuration.

9. The device of any preceding claim, wherein said average outer dimensions in said expanded configuration are said average outer dimensions when said stent graft is expanded in its unconstrained condition.

10. The device of any preceding claim, wherein said proximal end region, said intermediate region, and said distal end region each have an average outer dimension in said contracted configuration; and
wherein said average outer dimension of said proximal end region, said intermediate region, or said distal end region in said expanded configuration is at least 20% greater than the average outer dimensions of said same region in said contracted configuration;
preferably wherein said proximal end region, said intermediate region, and said distal end region each have an average outer dimension in said contracted configuration of 7 mm or less.

11. The device of any preceding claim, wherein said proximal end region of stent graft includes a transitional portion; and
wherein said transitional portion has a portion positioned along a central, longitudinal axis of said stent graft when said stent graft is in an expanded configuration
preferably wherein the transitional portion includes a helically-extending material.

12. The device of any preceding claim, wherein proximal end region of the stent graft is free of apices not having struts extending proximally therefrom.

13. A kit, comprising:
the stent graft (102) of any preceding claim, a catheter (103), and a sheath (106) within a sterily sealed package;
wherein said catheter (103) communicates with said first infusion/aspiration port (142) through said first infusion/aspiration lumen (140); and
wherein said stent graft (102) and said catheter (103) are positioned within a lumen of said sheath (106) with said stent in said contracted configuration.

14. The kit of claim 13, further comprising a first prefilled container of a first liquid that is capable of forming a hydrogel in vivo;
optionally wherein said first liquid forms a hydrogel when combined with a cross-linker; preferably wherein the kit further comprises a prefilled container of said cross-linker.

15. The kit of claim 13 or claim 14, further comprising a catheter segment extending through the proximal end region, intermediate region, and distal end region of the stent graft and defining a guidewire lumen;
optionally wherein said catheter comprises said catheter segment.

## Patentansprüche

1. Vorrichtung, umfassend:
eine Stentprothese (102), das sich von einem proximalen Endbereich (112) zu einem distalen Endbereich (114) erstreckt und einen Zwischenbereich (116) aufweist, der zwischen dem proximalen Endbereich (112) und dem distalen Endbereich (114) angeordnet ist;
wobei die Stentprothese (102) aus einer kontrahierten Konfiguration in eine expandierte Konfiguration konfigurierbar ist;
wobei die Stentprothese (102) ein mittiges Lumen (120) definiert, das sich vom proximalen Endbereich (112) zum distalen Endbereich (114) in der expandierten Konfiguration erstreckt und einen Prothesenmaterialabschnitt (122) im Zwischenbereich aufweist;
wobei der Prothesenmaterialabschnitt (122) eine nach innen weisende Seite (124), die dem mittigen Lumen (120) zugewandt ist, und eine nach außen weisende Seite (126), die vom mittigen Lumen (120) abgewandt ist, aufweist;
wobei der proximale Endbereich (112) und der distale Endbereich (114) jeweils eine durchschnittliche Außenabmessung aufweisen, wenn sich die Stentprothese (102) in der expandierten Konfiguration befindet;
wobei die durchschnittlichen Außenabmessungen in der expandierten Konfiguration des proximalen Endbereichs (112) und des distalen Endbereichs (114) jeweils größer als eine durchschnittliche Außenabmessung sind, die von dem Prothesenmaterial (122) des Zwischenabschnitts in der expandierten Konfiguration definiert wird; und
eine erste Infusions-/Aspirationsöffnung (142) in Fluidverbindung mit einem ersten Infusions-/Aspirationslumen (140) ;
wobei die erste Infusions-/Aspirationsöffnung (142) auf der nach außen weisenden Seite (126) des Prothesenmaterials (122) mündet und sich das erste Infusions-/Aspirationslumen (140) proximal von der ersten Infusions-/Aspirationsöffnung (142) entlang des proximalen Endbereichs (112) der Stentprothese erstreckt, und sich entlang einer nach außen weisenden Seite eines Prothesenmaterials der Stentprothese im proximalen Endbereich (112) erstreckt.

2. Vorrichtung nach Anspruch 1, wobei die Stentprothese einen Stent mit einer nach außen weisenden Seite, die vom mittigen Lumen der Stentprothese abgewandt ist, und mit einer nach innen weisenden Seite, die dem mittigen Lumen zugewandt ist, aufweist; und
wobei sich das erste Infusions-/Aspirationslumen entlang der nach innen weisenden Seite des Stents im proximalen Endbereich erstreckt.

3. Vorrichtung nach einem der vorhergehenden Ansprüche, wobei der Stent selbstexpandierend ist.

4. Vorrichtung nach einem der vorhergehenden Ansprüche, wobei eine zweite Infusions-/Aspirationsöffnung mit einem zweiten Infusions-/Aspirationslumen in Fluidverbindung steht; und
wobei die zweite Infusions-/Aspirationsöffnung zur nach außen weisenden Seite des Prothesenmaterials mündet;
vorzugsweise wobei die Infusions-/Aspirationsöffnungen gleichmäßig um den Zwischenbereich der Stentprothese verteilt sind.

5. Vorrichtung nach Anspruch 4, wobei das erste Lumen als ein Infusionslumen ausgelegt ist, wobei die erste Öffnung als eine Infusionsöffnung ausgelegt ist, das zweite Lumen als ein Aspirationslumen ausgelegt ist und die zweite Öffnung als eine Aspirationsöffnung ausgelegt ist.

6. Vorrichtung nach einem der vorhergehenden Ansprüche, wobei das erste Infusions-/Aspirationslumen von einem Katheter definiert wird.

7. Vorrichtung nach einem der vorhergehenden Ansprüche, wobei das erste Infusions-/Aspirationslumen zwischen dem Prothesenmaterial und einem zweiten Prothesenmaterial angeordnet ist; und
wobei das zweite Prothesenmaterial mit dem Prothesenmaterial verbunden ist.

8. Vorrichtung nach einem der vorhergehenden Ansprüche, wobei sich das Infusions-/Aspirationslumen in einer proximalen Richtung durch eine Hülle erstreckt; und
wobei die Hülle eine Größe und Konfiguration zum Enthalten der Stentprothese in der kontrahierten Konfiguration aufweist.

9. Vorrichtung nach einem der vorhergehenden Ansprüche, wobei die durchschnittlichen Außenabmessungen in der expandierten Konfiguration die durchschnittlichen Außenabmessungen sind, wenn die Stentprothese in seinen nicht eingezwängten Zustand aufgeweitet wird.

10. Vorrichtung nach einem der vorhergehenden Ansprüche, wobei der proximale Endbereich, der Zwischenbereich und der distale Endbereich jeweils ein durchschnittliche Außenabmessung in der kontrahierten Konfiguration aufweisen; und
wobei die durchschnittliche Außenabmessung des proximalen Endbereichs, des Zwischenbereichs oder des distalen Endbereichs in der expandierten Konfiguration mindestens 20% größer als die durchschnittlichen Außenabmessungen desselben Bereichs in der kontrahierten Konfiguration ist;
vorzugsweise wobei der proximale Endbereich, der Zwischenbereich und der distale Endbereich jeweils eine durchschnittliche Außenabmessung in der kontrahierten Konfiguration von 7 mm oder weniger aufweisen.

11. Vorrichtung nach einem der vorhergehenden Ansprüche, wobei der proximale Endbereich der Stentprothese einen Übergangsabschnitt aufweist; und
wobei der Übergangsabschnitt einen Abschnitt aufweist, der entlang einer Mittellängsachse der Stentprothese angeordnet ist, wenn sich die Stentprothese in einer expandierten Konfiguration befindet,
vorzugsweise wobei der Übergangsabschnitt ein sich spiralförmig erstreckendes Material aufweist.

12. Vorrichtung nach einem der vorhergehenden Ansprüche, wobei der proximale Endbereich der Stentprothese keine Spitzen aufweist, die keine sich proximal davon erstreckenden Streben aufweisen.

13. Kit, umfassend:
die Stentprothese (102) nach einem der vorhergehenden Ansprüche, einen Katheter (103) und eine Hülle (106) innerhalb einer steril verschlossenen Verpackung;
wobei der Katheter (103) mit der ersten Infusions-/Aspirationsöffnung (142) durch das erste Infusions-/Aspirationslumen (140) in Verbindung steht; und
wobei die Stentprothese (102) und der Katheter (103) in einem Lumen der Hülle (106) angeordnet sind, wobei sich der Stent in der kontrahierten Konfiguration befindet.

14. Kit nach Anspruch 13, ferner umfassend einen ersten vorgefüllten Behälter mit einer ersten Flüssigkeit, die in vivo ein Hydrogel bilden kann;
fakultativ wobei die erste Flüssigkeit ein Hydrogel bildet, wenn sie mit einem Vernetzungsmittel kombiniert wird; vorzugsweise wobei das Kit ferner einen vorgefüllten Behälter mit dem Vernetzungsmittel umfasst.

15. Kit nach Anspruch 13 oder Anspruch 14, ferner umfassend ein Kathetersegment, das sich durch den proximalen Endbereich, den Zwischenbereich und den distalen Endbereich der Stentprothese erstreckt und ein Führungsdrahtlumen definiert;
fakultativ wobei der Katheter das Kathetersegment umfasst.

## Revendications

1. Dispositif, comprenant :
un greffon de stent (102) s'étendant depuis une région d'extrémité proximale (112) vers une région d'extrémité distale (114) et possédant une région intermédiaire (116) positionnée entre ladite région d'extrémité proximale (112) et ladite région d'extrémité distale (114) ;
ledit greffon de stent (102) étant configurable depuis une configuration contractée vers une configuration déployée ;
ledit greffon de stent (102) définissant une lumière centrale (120) s'étendant depuis la région d'extrémité proximale (112) vers la région d'extrémité distale (114) dans ladite configuration déployée et possédant une partie matériau de greffon (122) dans ladite région intermédiaire ;
ladite partie matériau de greffon (122) possédant un côté tourné vers l'intérieur (124) qui fait face à ladite lumière centrale (120) et un côté tourné vers l'extérieur (126) qui tourne le dos à ladite lumière centrale (120) ;
ladite région d'extrémité proximale (112) et ladite région d'extrémité distale (114) ayant chacune une dimension externe moyenne lorsque ledit greffon de stent (102) est dans ladite configuration déployée ;
lesdites dimensions externes moyennes, dans ladite configuration déployée, de ladite région d'extrémité proximale (112) et de ladite région d'extrémité distale (114) étant chacune supérieures à une dimension externe moyenne définie par ledit matériau de greffon (122) de ladite région intermédiaire dans ladite configuration déployée ; et
un premier orifice de perfusion/aspiration (142) en communication fluidique avec une première lumière de perfusion/aspiration (140) ;
ledit premier orifice de perfusion/aspiration (142) donnant sur ledit côté tourné vers l'extérieur (126) dudit matériau de greffon (122), et ladite première lumière de perfusion/aspiration (140) s'étendant proximalement depuis ledit premier orifice de perfusion/aspiration (142) le long de ladite région d'extrémité proximale (112) dudit greffon de stent, et s'étendant le long d'un côté tourné vers l'extérieur d'un matériau de greffon dudit greffon de stent dans ladite région d'extrémité proximale (112).

2. Dispositif selon la revendication 1, dans lequel ledit greffon de stent comprend un stent possédant un côté tourné vers l'extérieur qui tourne le dos à la lumière centrale du greffon de stent et un côté tourné vers l'intérieur qui fait face à la lumière centrale ; et
dans lequel ladite première lumière de perfusion/aspiration s'étend le long dudit côté tourné vers l'intérieur dudit stent dans ladite région d'extrémité proximale.

3. Dispositif selon l'une quelconque des revendications précédentes, dans lequel ledit stent est autodéployable.

4. Dispositif selon l'une quelconque des revendications précédentes, dans lequel un second orifice de perfusion/aspiration est en communication fluidique avec une seconde lumière de perfusion/aspiration ; et
dans lequel ledit second orifice de perfusion/aspiration donne sur ledit côté tourné vers l'extérieur dudit matériau de greffon ;
de préférence dans lequel lesdits orifices de perfusion/aspiration sont répartis uniformément autour de ladite région intermédiaire dudit greffon de stent.

5. Dispositif selon la revendication 4, dans lequel ladite première lumière est conçue en tant que lumière de perfusion, ledit premier orifice est conçu en tant qu'orifice de perfusion, ladite seconde lumière est conçue en tant que lumière d'aspiration et ledit second orifice est conçu en tant qu'orifice d'aspiration.

6. Dispositif selon l'une quelconque des revendications précédentes, dans lequel ladite première lumière de perfusion/aspiration est définie par un cathéter.

7. Dispositif selon l'une quelconque des revendications précédentes, dans lequel ladite première lumière de perfusion/aspiration est positionnée entre ledit matériau de greffon et un second matériau de greffon ; et
dans lequel ledit second matériau de greffon est lié audit matériau de greffon.

8. Dispositif selon l'une quelconque des revendications précédentes, dans lequel ladite lumière de perfusion/aspiration s'étend dans une direction proximale à travers une gaine ; et
dans lequel ladite gaine est dimensionnée et conçue pour contenir ledit greffon de stent dans ladite configuration contractée.

9. Dispositif selon l'une quelconque des revendications précédentes, dans lequel lesdites dimensions externes moyennes dans ladite configuration déployée sont lesdites dimensions externes moyennes lorsque ledit greffon de stent est déployé dans son état non contraint.

10. Dispositif selon l'une quelconque des revendications précédentes, dans lequel ladite région d'extrémité proximale, ladite région intermédiaire et ladite région d'extrémité distale ont chacune une dimension externe moyenne dans ladite configuration contractée ; et
dans lequel ladite dimension externe moyenne de ladite région d'extrémité proximale, ladite région intermédiaire ou ladite région d'extrémité distale dans ladite configuration déployée est au moins 20 % plus grande que les dimensions externes moyennes de ladite même région dans ladite configuration déployée ;
de préférence dans lequel ladite région d'extrémité proximale, ladite région intermédiaire et ladite région d'extrémité distale ont chacune une dimension externe moyenne dans ladite configuration contractée inférieure ou égale à 7 mm.

11. Dispositif selon l'une quelconque des revendications précédentes, dans lequel ladite région d'extrémité proximale du greffon de stent comprend une partie de transition ; et
dans lequel ladite partie de transition possède une partie positionnée le long d'un axe longitudinal central dudit greffon de stent lorsque ledit greffon de stent est dans une configuration déployée
de préférence dans lequel la partie de transition comprend un matériau s'étendant hélicoïdalement.

12. Dispositif selon l'une quelconque des revendications précédentes, dans lequel la région d'extrémité proximale du greffon de stent est dépourvue de sommets ne possédant pas d'entretoises s'étendant proximalement depuis ceux-ci.

13. Kit, comprenant :
le greffon de stent (102) selon l'une quelconque des revendications précédentes, un cathéter (103) et une gaine (106) dans un emballage stérile fermé hermétiquement ;
dans lequel ledit cathéter (103) communique avec ledit premier orifice de perfusion/aspiration (142) par l'intermédiaire de ladite première lumière de perfusion/aspiration (140) ; et
dans lequel ledit greffon de stent (102) et ledit cathéter (103) sont positionnés dans une lumière de ladite gaine (106), ledit stent étant dans ladite configuration contractée.

14. Kit selon la revendication 13, comprenant en outre un premier contenant prérempli d'un premier liquide qui est susceptible de former un hydrogel in vivo ;
éventuellement dans lequel ledit premier liquide forme un hydrogel lorsqu'il est associé à un agent de réticulation ; de préférence, le kit comprenant en outre un contenant prérempli dudit agent de réticulation.

15. Kit selon la revendication 13 ou la revendication 14, comprenant en outre un segment de cathéter s'étendant à travers la région d'extrémité proximale, la région intermédiaire et la région d'extrémité distale du greffon de stent et définissant une lumière de guide-fil ;
éventuellement dans lequel ledit cathéter comprend ledit segment de cathéter.
